# EUROPÄISCHE PATENTSCHRIFT

(11) **EP 2 089 153 B1**
(45) Veröffentlichungstag und Bekanntmachung des Hinweises auf die Patenterteilung: **02.05.2012**
(21) Anmeldenummer: 07821749.4
(22) Anmeldetag: 24.10.2007
(51) Int. Cl.: B01J 35/00, C07C 63/38, B01J 31/22, B01J 20/28, C07C 51/41, B01J 20/22, B01J 20/26, C01B 3/00, F17C 11/00, B01J 31/16

(54) **ALUMINIUM-NAPHTHALINDICARBOXYLAT ALS PORÖSES METALLORGANISCHES GERÜSTMATERIAL**
ALUMINUM NAPHTHALENEDICARBOXYLATE AS A POROUS ORGANOMETALLIC FRAMEWORK MATERIAL
ALUMINIUM-NAPHTALINEDICARBOXYLATE EN TANT QUE MATÉRIAU SQUELETTE MÉTALLO-ORGANIQUE POREUX

(30) Priorität: 30.10.2006 EP 06123200
(43) Veröffentlichungstag der Anmeldung: 19.08.2009
(73) Patentinhaber: BASF SE, 67056 Ludwigshafen (DE)
(72) Erfinder: SCHUBERT, Markus, 67063 Ludwigshafen (DE); MÜLLER, Ulrich, 67435 Neustadt (DE); MARX, Stefan, 46049 Oberhausen (DE); KIENER, Christoph, 67256 Weisenheim am Sand (DE); BROWN, James, Reuben, 68159 Mannheim (DE); KRENNRICH, Gerhard, 67227 Frankenthal (DE)
(86) Internationale Anmeldenummer: PCT/EP2007/061386
(87) Internationale Veröffentlichungsnummer: WO 2008/052916

(56) Entgegenhaltungen:
- EP-A- 1 702 925
- WO-A-03/064030
- WO-A-2007/023134
- WO-A-2007/131948
- JP-A- 59 155 333
- LOISEAU T ET AL: "A Rationale for the Large Breathing of the Porous Aluminum Terephthalate (MIL-53) Upon Hydration" CHEMISTRY - A EUROPEAN JOURNAL, VCH PUBLISHERS, US, Bd. 10, Nr. 6, 15. März 2004 (2004-03-15), Seiten 1373-1382, XP002456416 ISSN: 0947-6539
- LOISEAU T ET AL: "Hydrothermal synthesis and crystal structure of a new three-dimensional aluminum-organic framework MIL-69 with 2,6-naphthalenedicarboxylate (ndc), Al(OH)(ndc).H2O" COMPTES RENDUS CHIMIE, EDITIONS SCIENTIFIQUES ET MEDICALES ELSEVIER, Bd. 8, Nr. 3-4, März 2005 (2005-03), Seiten 765-772, XP004858426 ISSN: 1631-0748
- I. SENKOVSKA, S. KASKEL: "Solvent-Induced Pore-size Adjustment in the Metal-Organic Framework [Mg3(ndc)3(dmf)4] (ndc = naphthalenedicarboxylate)" EUR. J. INORG. CHEM., Bd. 2006, Nr. 122, 25. September 2006 (2006-09-25), Seiten 4564-4569, XP002467340 Weinheim
- S. BOURELLY, P.L. LLEWELLYN, C. SERRE, F. MILLANGE, T. LOISEAU, G. FEREY: "Different Adsorption Behaviors of Methane and Carbon Dioxide in the Isotypic nanoporous Metal Terephthalates MIL-53 and Mil-47" J. AM. CHEM. SOC., Bd. 2005, Nr. 127, 13. September 2005 (2005-09-13), Seiten 13519-13521, XP002467341

## Beschreibung

Die vorliegende Erfindung betrifft ein poröses metallorganisches Gerüstmaterial, Formkörper dieses enthaltend, Verfahren zur Herstellung des Gerüstmaterials sowie die Verwendung des besagten Gerüstmaterials oder Formkörpers für die Speicherung, Abtrennung, kontrollierte Abgabe, chemische Umsetzung oder als Träger.

Poröse metallorganische Gerüstmaterialien sind im Stand der Technik bekannt. Sie zeichnen sich insbesondere durch ihre Porosität aus und sind häufig vergleichbaren Anwendungen zuführbar, welche von anorganischen Zeolithen bekannt sind.

Metallorganische Gerüstmaterialien enthalten üblicherweise eine an ein Metallion koordinativ gebundene, mindestens zweizähnige organische Verbindung, die gemeinsam mit dem Metallion das Gerüst des metallorganischen Gerüstmaterials darstellt. Dokument WO 03/064030 offenbart ebenfalls Gerüst materialien für die Speicherung von Gasen.

Die geeignete Wahl von Metall und/oder organischer Verbindung ermöglicht eine Optimierung für das gewünschte Anwendungsgebiet. Hierbei kann beispielsweise die Wahl der organischen Verbindung Einfluss auf die Porenverteilung nehmen. Darüber hinaus kann das Metall einen Beitrag bei Adsorptionsvorgängen liefern.

Es existiert also ein stetiger Bedarf, spezielle metallorganische Gerüstmaterialien bereitzustellen, die insbesondere außergewöhnliche Eigenschaften aufweisen, welche auf die Wahl des Metalls sowie der organischen Verbindung zurückzuführen sind.

Als ein interessantes Metall kann Aluminium genannt werden, da aufgrund starker Koordinationsbindungen vergleichsweise robuste metallorganische Gerüstmaterialien erhalten werden können. Zudem ist das Al³⁺-lon aufgrund seiner oktaedrischen Koordination prinzipiell geeignet, dreidimensionale Gerüstverbindungen aufzubauen. Weiterhin sind die als Einsatzstoff verwendbaren Salze des Aluminiums gut zugänglich und preiswert.

Als eine interessante, zweizähnige organische Verbindung kann 2,6-Naphthalindicarbonsäure genannt werden. Aufgrund des Naphthalingerüstes sowie der Position der beiden Carbonsäurefunktionalitäten können die einzelnen Metallzentren im porösen metallorganischen Gerüstmaterial vergleichsweise stark beabstandet werden, wobei mit Hilfe des starren Naphthalingerüstes das metallorganische Gerüstmaterial als solches ebenfalls vergleichsweise rigide und robust ist. 2,6-Naphthalindicarbonsäure selbst ist chemisch und thermisch vergleichweise stabil und wie die Salze des Aluminiums in großer Menge verfügbar.

T. Loiseau et al., C.R. Chimie 8 (2005), 765-772, beschreiben die hydrothermale Synthese und Kristallstruktur des dreidimensional aufgebauten Aluminium-organischen Gerüstmaterials "MIL-69", welches aus Aluminiumionen und 2,6-Naphthalindicarboxylat aufgebaut ist. Hierbei werden die Kristallstruktur sowie weitere physikalische Eigenschaften des Gerüstmaterials untersucht.

Dabei wird für die Struktur "MIL-69" eine stark verzerrte, aber ansonsten analoge Struktur zu "MIL-53" (Aluminiumterephthalat) gefunden, wobei jedoch das typischerweise in dieser Struktur eingelagerte Wassermolekül ohne Änderung der Struktur entfernt werden kann.

Eine Reproduktion des beschriebenen Gerüstmaterials ergibt jedoch vergleichsweise geringe spezifische Oberflächen, so dass deren Eignung für Anwendungen wie die Speicherung oder Trennung von Gasen vergleichsweise gering ist.

Es besteht daher ein Bedarf an alternativen Aluminium-Naphthalindicarboxylat-Gerüstmaterialien, die überlegene Eigenschaften insbesondere in Bezug auf die Speicherung und Trennung von Gasen aufweisen.

Eine Aufgabe der vorliegenden Erfindung liegt somit in der Bereitstellung solcher Gerüstmaterialien.

Die Aufgabe wird gelöst durch ein Verfahren zur Herstellung eines porösen metallorganischen Gerüstmaterial, enthaltend eine an ein Metallion koordinativ gebundene, zweizähnige organische Verbindung, wobei das Metallion Al^{III} und die zweizähnige organische Verbindung 2,6-Naphthalindicarboxylat ist, dadurch gekennzeichnet, dass das Röntgendiffraktogramm (XRD) des Gerüstmaterials im Bereich von 6,5° < 2Θ < 7,5° einen ersten Reflex und im Bereich von 13,8° < 2Θ < 15,0° einen zweiten Reflex aufweist, wobei in Bezug auf die Fläche aller Reflexe im Bereich von 2°< 2Θ < 70° die Fläche des ersten Reflexes am grössten und die Fläche des zweiten Reflexes am zweitgrössten ist und wobei die Summe der Flächen von erstem und zweitem Reflex in Bezug auf die Gesamtfläche aller Reflexe im Bereich von 2° < 2Θ < 70° mindestens 50 % ergibt, den Schritt enthaltend die Umsetzung eines Reaktionsgemisches enthaltend eine Aluminiumverbindung, 2,6- Naphthalindicarbonsäure oder eines ihrer Salze und N,N-Dimethylformamid als organisches Lösemittel bei Temperaturen im Bereich von 100 °C bis 200 °C unter Rühren, wobei die Summe der Anteile an der Aluminiumverbindung und der 2,6-Naphthalindicarbonsäure oder einem ihrer Salze weniger als 20 Gew.-% bezogen auf das Gesamtgewicht des Reaktionsgemisches beträgt und ein poröses metallorganisches Gerüstmaterial gemäß Anspruch 3 und dessen Verwendung gemäß Anspruch 8.

Es wurde gefunden, dass durch Variation der Herstellbedingungen es möglich ist, ein neues metallorganisches Gerüstmaterial zu erhalten, das wie die im Stand der Technik bekannte Struktur "MIL-69" aus Aluminiumionen und 2,6-Naphthalindicarboxylat aufgebaut ist, jedoch ein zu diesem unterschiedliches Röntgendiffraktogramm aufweist und darüber hinaus über eine vergleichsweise hohe spezifische Oberfläche verfügt.

Hierbei hat sich insbesondere gezeigt, dass die Auswahl des Gewichtsanteils der Edukte (Aluminium und 2,6-Naphthalindicarboxylat) im Verhältnis zum Gesamtgewicht des Reaktionsgemisches dahingehend, dass ein bestimmter Wert unterschreiten wird, die Bildung der neuen Struktur ermöglicht.

Wie bereits oben ausgeführt wurde, unterscheidet sich die neue Struktur deutlich in ihrem Röntgendiffraktogramm.

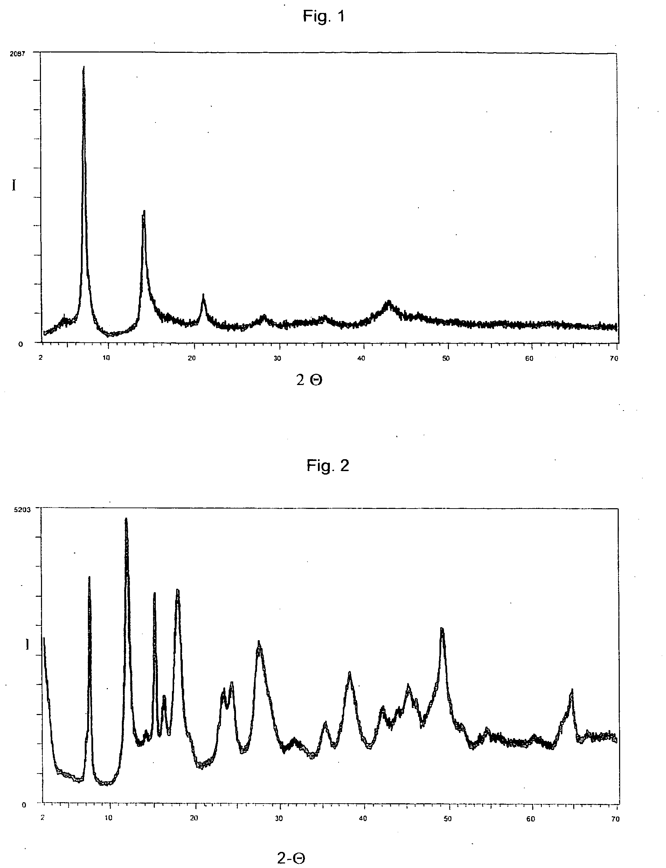

In Fig. 1 ist das Röntgendiffraktogramm des erfindungsgemäßen porösen metallorganischen Gerüstmaterials gezeigt. Hierbei ist - wie bei allen in den Figuren dargestellten Diffraktogrammen - die Intensität I (Lin(Counts)) als Funktionen der 2 Theta-Skale (2θ) dargestellt.

Demgegenüber zeigt Fig. 2 die aus dem Stand der Technik bekannte Gerüststruktur "MIL-69".

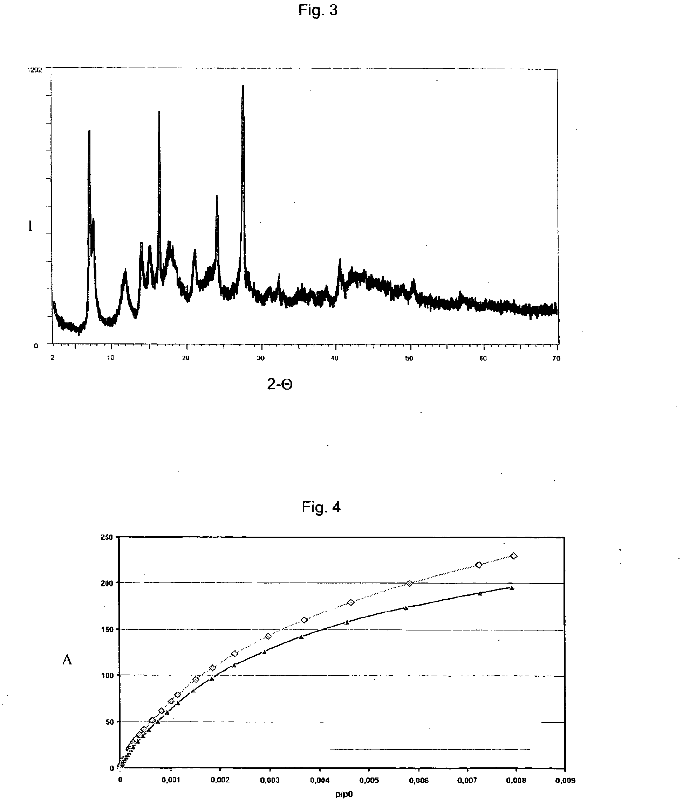

Anhand der deutlich unterschiedlichen Röntgendiffraktogramme können die beiden Strukturen voneinander unterschieden werden. Es ist ebenso möglich, dass das erfindungsgemäße poröse metallorganische Gerüstmaterial in Mischung mit dem aus dem Stand der Technik vorliegt, wobei entsprechend eine Überlagerung beider Diffraktogramme erfolgt. Ein solches Beispiel ist in Fig. 3 gezeigt.

Die Struktur des erfindungsgemäßen porösen metallorganischen Gerüstmaterials kann insbesondere daran erkannt werden, dass das Röntgendiffraktogramm (XRD) im Bereich von 6,5° < 2Θ < 7,5° einen ersten Reflex und im Bereich von 13,8° < 2Θ < 15,0° einen zweiten Reflex aufweist, wobei in Bezug auf die Fläche aller Reflexe im Bereich von 2° < 2Θ < 70° die Fläche des ersten Reflexes am größten und die Fläche des zweiten Reflexes am zweitgrößten ist und wobei die Summe der Flächen von erstem und zweitem Reflex in Bezug auf die Gesamtfläche aller Reflexe im Bereich von 2° < 2Θ < 70° mindestens 50 % ergibt.

Vorzugsweise liegt der erste Reflex im Bereich von 6,7° < 2Θ < 7,3° und der zweite Reflex im Bereich von 13,9 ° < 2Θ < 14,8°, insbesondere ein Bereich von 14,0° < 2 Θ < 14,5°.

Hierbei kann das Diffraktogramm wie folgt ermittelt werden: Die Probe wird als Pulver in den Probenbehälter eines kommerziell erhältlichen Geräts (Siemens D-5000 Diffraktometer oder Bruker D8-Advance) eingebaut. Als Strahlungsquelle wird Cu-Kα-Strahlung mit variablen Primär- und Sekundärblenden und Sekundärmonochromator benutzt. Die Detektion des Signals erfolgt über einen Szintillations- (Siemens) oder Solex-Halbleiter Detektor (Bruker). Der Messbereich für 2θ wird typischerweise zwischen 2° und 70 ° gewählt. Der Winkelschritt beträgt 0,02°, die Messzeit pro Winkelschritt typischerweise 2-4s. Bei der Auswertung werden Reflexe durch eine wenigstens 3-fach höhere Signalstärke vom Grundrauschen unterschieden. Die Flächenanalyse kann manuell erfolgen, indem an die einzelnen Reflexe eine Basislinie angelegt wird. Alternativ können Programme wie zum Beispiel "Topas-Profile" der Fa. Bruker eingesetzt werden, wobei die Untergrundanpassung dann bevorzugt über ein Polynom 1. Grades in der Software automatisch erfolgt.

Die Struktur des erfindungsgemäßen Gerüstmaterials weist vorzugsweise eine eindimensionale Kanalstruktur auf, bei der lineare Ketten aus Al^{III}-Ionen und OH-Gruppen über das organische 2,5-Naphthalindicarboxylat zu einer dreidimensionalen, orthorhombischen Struktur verbrückt werden, wobei der Öffnungswinkel α (AI-AI-AI) im Bereich von 78° bis 90°, bevorzugt im Bereich von 80° bis 90°, mehr bevorzugt bei 82,5° ± 1,5° oder bei 88,5° ± 1,5° liegt.

Weiterhin weist die Struktur des erfindungsgemäßen Gerüstmaterials eine Gitterkonstante b (Höhe der Einheitszelle) von 16 bis 18,5 Å auf.

Das erfindungsgemäße metallorganische Gerüstmaterial kann pulverförmig beziehungsweise als Agglomerat vorliegen.

Das erfindungsgemäße poröse metallorganische Gerüstmaterial kann als solches in Pulverform verwendet werden oder es wird in einen Formkörper umgewandelt.

Dementsprechend ist ein weiterer Aspekt der vorliegenden Erfindung, dass das erfindungsgemäße poröse metallorganische Gerüstmaterial als Pulver vorliegt.

Ein weiterer Aspekt der vorliegenden Erfindung ist demzufolge ein Formkörper enthaltend das erfindungsgemäße poröse metallorganische Gerüstmaterial.

Die Herstellung von Formkörpern aus metallorganischen Gerüstmaterialien ist beispielsweise in WO-A 03/102000 beschrieben.

Bevorzugte Verfahren zur Herstellung von Formkörpern sind hierbei die Verstrangung oder Tablettierung. Bei der Formkörperherstellung kann das Gerüstmaterial weitere Materialien, wie beispielsweise Binder, Gleitmittel oder andere Additive aufweisen, welche während der Herstellung hinzugesetzt werden. Ebenso ist es denkbar, dass das Gerüstmaterial weitere Bestandteile aufweist, wie zum Beispiel Absorbentien wie Aktivkohle oder dergleichen.

Hinsichtlich der möglichen Geometrien der Formkörper existieren im Wesentlichen keine Beschränkungen. Beispielsweise sind unter anderem Pellets wie beispielsweise scheibenförmige Pellets, Pillen, Kugeln, Granulat, Extrudate wie beispielsweise Stränge, Waben, Gitter oder Hohlkörper zu nennen.

Zur Herstellung dieser Formkörper sind grundsätzlich sämtliche geeigneten Verfahren möglich. Es sind insbesondere folgende Verfahrensführungen bevorzugt:
- Kneten/Kollern des Gerüstmaterials allein oder zusammen mit mindestens einem Bindemittel und/oder mindestens einem Anteigungsmittel und/oder mindestens
   einer Templatverbindung unter Erhalt eines Gemisches; Verformen des erhaltenen Gemisches mittels mindestens einer geeigneten Methode wie beispielsweise Extrudieren; Optional Waschen und/oder Trocknen und/oder Calcinieren des Extrudates; Optional Konfektionieren.
- Tablettieren zusammen mit mindestens einem Bindemittel und/oder anderem Hilfsstoff.
- Aufbringen des Gerüstmaterials auf mindestens ein gegebenenfalls poröses Trägermaterial. Das erhaltene Material kann dann gemäß der vorstehend beschriebenen Methode zu einem Formkörper weiterverarbeitet werden.
- Aufbringen des Gerüstmaterials auf mindestens ein gegebenenfalls poröses Substrat.

Kneten/Kollern und Verformen kann gemäß eines jeden geeigneten Verfahrens erfolgen, wie beispielsweise in Ullmanns Enzyklopädie der Technischen Chemie, 4. Auflage, Band 2, S. 313 ff. (1972) beschrieben.

Beispielsweise kann das Kneten/Kollern und/oder Verformen mittels einer Kolbenpresse, Walzenpresse in Anwesenheit oder Abwesenheit mindestens eines Bindermaterials, Compoundieren, Pelletieren, Tablettieren, Extrudieren, Co-Extrudieren, Verschäumen, Verspinnen, Beschichten, Granulieren, bevorzugt Sprühgranulieren, Versprühen, Sprühtrocknen oder einer Kombination aus zwei oder mehr dieser Methoden erfolgen.

Ganz besonders bevorzugt werden Pellets und/oder Tabletten hergestellt.

Das Kneten und/oder Verformen kann bei erhöhten Temperaturen wie beispielsweise im Bereich von Raumtemperatur bis 300 °C und/oder bei erhöhtem Druck wie beispielsweise im Bereich von Normaldruck bis hin zu einigen hundert bar und/oder in einer Schutzgasatmosphäre wie beispielsweise in Anwesenheit mindestens eines Edelgases, Stickstoff oder einem Gemisch aus zwei oder mehr davon erfolgen.

Das Kneten und/oder Verformen wird gemäß einer weiteren Ausführungsform unter Zugabe mindestens eines Bindemittels durchgeführt, wobei als Bindemittel grundsätzlich jede chemische Verbindung eingesetzt werden kann, die die zum Kneten und/oder Verformen gewünschte Viskosität der zu verknetenden und/oder verformenden Masse gewährleistet. Demgemäß können Bindemittel im Sinne der vorliegenden Erfindung sowohl Viskositätserhöhende als auch Viskositätserniedrigende Verbindungen sein.

Als unter anderem bevorzugte Bindemittel sind beispielsweise Aluminiumoxid oder Aluminiumoxid enthaltende Binder, wie sie beispielsweise in der WO 94/29408 beschrieben sind, Siliciumdioxid, wie es beispielsweise in der EP 0 592 050 A1 beschrieben ist, Mischungen ais Siliciumdioxid und Aluminiumoxid, wie sie beispielsweise in der WO 94/13584 beschrieben sind, Tonminerale, wie sie beispielsweise in der JP 03-037156 A beschrieben sind, beispielsweise Montmorillonit, Kaolin, Bentonit, Halloysit, Dickit, Nacrit und Anauxit, Alkoxysilane, wie sie beispielsweise in der EP 0 102 544 B1 beschrieben sind, beispielsweise Tetraalkoxysilane wie beispielsweise Tetramethoxysilan, Tetraethoxysilan, Tetrapropoxysilan, Tetrabutoxysilan, oder beispielsweise Trialkoxysilane wie beispielsweise Trimethoxysilan, Triethoxysilan, Tripropoxysilan, Tributoxysilan, Alkoxytitanate, beispielsweise Tetraalkoxytitanate wie beispielsweise Tetramethoxytitanat, Tetraethoxytitanat, Tetrapropoxytitanat, Tetrabutoxytitanat, oder beispielsweise Trialkoxytitanate wie beispielsweise Trimethoxytitanat, Triethoxytitanat, Tripropoxytitanat, Tributoxytitanat, Alkoxyzirkonate, beispielsweise Tetraalkoxyzirkonate wie beispielsweise Tetramethoxyzirkonat, Tetraethoxyzirkonat, Tetrapropoxyzirkonat, Tetrabutoxyzirkonat, oder beispielsweise Trialkoxyzirkonate wie beispielsweise Trimethoxyzirkonat, Triethoxyzirkonat, Tripropoxyzirkonat, Tributoxyzirkonat, Silikasole, amphiphile Substanzen und/oder Graphite zu nennen.

Als viskositätssteigernde Verbindung kann beispielsweise auch, gegebenenfalls zusätzlich zu den oben genannten Verbindungen, eine organische Verbindung und/oder ein hydrophiles Polymer wie beispielsweise Cellulose oder ein Cellulosederivat wie beispielsweise Methylcellulose und/oder ein Polyacrylat und/oder ein Polymethacrylat und/oder ein Polyvinylalkohol und/oder ein Polyvinylpyrrolidon und/oder ein Polyisobuten und/oder ein Polytetrahydrofuran und/oder ein Polyethylenoxid eingesetzt werden.

Als Anteigungsmittel kann unter anderem bevorzugt Wasser oder mindestens ein Alkohol wie beispielsweise ein Monoalkohol mit 1 bis 4 C-Atomen wie beispielsweise Methanol, Ethanol, n-Propanol, iso-Propanol, 1-Butanol, 2-Butanol, 2-Methyl-1-propanol oder 2-Methyl-2-propanol oder ein Gemisch aus Wasser und mindestens einem der genannten Alkohole oder ein mehrwertiger Alkohol wie beispielsweise ein Glykol, bevorzugt ein wassermischbarer mehrwertiger Alkohol, allein oder als Gemisch mit Wasser und/oder mindestens einem der genannten einwertigen Alkohole eingesetzt werden.

Weitere Additive, die zum Kneten und/oder Verformen eingesetzt werden können, sind unter anderem Amine oder Aminderivate wie beispielsweise Tetraalkylammonium-Verbindungen oder Aminoalkohole und Carbonat enthaltende Verbindungen wie etwa Calciumcarbonat. Solche weiteren Additive sind etwa in der EP 0 389 041 A1, der EP 0 200 260 A1 oder der WO 95/19222 beschrieben.

Die Reihenfolge der Additive wie Templatverbindung, Binder, Anteigungsmittel, viskositätssteigernde Substanz beim Verformen und Kneten ist grundsätzlich nicht kritisch.

Gemäß einer weiteren bevorzugten Ausführungsform wird der gemäß Kneten und/oder Verformen erhaltene Formkörper mindestens einer Trocknung unterzogen, die im Allgemeinen bei einer Temperatur im Bereich von 25 bis 500 °C, bevorzugt im Bereich von 50 bis 500 °C und besonders bevorzugt im Bereich von 100 bis 350 °C durchgeführt wird. Ebenso ist es möglich, im Vakuum oder unter Schutzgasatmosphäre oder durch Sprühtrocknung zu trocknen.

Gemäß einer besonders bevorzugten Ausführungsform wird im Rahmen dieses Trocknungsvorgangs mindestens eine der als Additive zugesetzten Verbindungen zumindest teilweise aus dem Formkörper entfernt.

Das erfindungsgemäße metallorganische Gerüstmaterial enthält Poren, insbesondere Mikro- und/oder Mesoporen. Mikroporen sind definiert als solche mit einem Durchmesser von 2 nm oder kleiner und Mesoporen sind definiert durch einen Durchmesser im Bereich von 2 bis 50 nm (Pure & Appl. Chem. 57 (1985) 603-619). Die Anwesenheit von Mikro- und/oder Mesoporen kann mit Hilfe von Sorptionsmessungen überprüft werden, wobei diese Messungen die Aufnahmekapazität der metallorganischen Gerüstmaterialien für Stickstoff bei 77 Kelvin (nach Langmuir) gemäß DIN 66131 und/oder DIN 66134 bestimmt.

Bevorzugt liegen die Poren in Form eindimensionaler Kanäle mit einem Durchmesser von mehr als 10 aber weniger als 25 Angström, besonders bevorzugt von 15 bis 20, insbesondere von 16 bis 18 Å vor.

Vorzugsweise beträgt die spezifische Oberfläche - berechnet nach dem Langmuir-Modell (DIN 66131, 66134) für das erfindungsgemäße metallorganische Gerüstmaterial in Pulverform mindestens 1000 m²/g, mehr bevorzugt mindestens 1200 m²/g, mehr bevorzugt mindestens 1400 m²/g, weiter mehr bevorzugt mindestens 1600 m²/g, weiter mehr bevorzugt mindestens 1800 m²/g und besonders bevorzugt mindestens 1950 m²/g_{.}

Formkörper aus dem erfindungsgemäßen metallorganischen Gerüstmaterial können eine niedrigere spezifische Oberfläche besitzen; vorzugsweise jedoch mindestens 500 m²/g, mehr bevorzugt mindestens 600 m²/g, weiter mehr bevorzugt mindestens 700 m²/g, insbesondere mindestens 800 m²/g.

Als organische Komponente des erfindungsgemäßen porösen metallorganischen Gerüstmaterials dient 2,6-Naphthalindicarbonsäure, die mit einer Aluminiumverbindung umgesetzt werden kann. Ebenso ist es möglich, Derivate der 2,6-Naphthalindicarbonsäure einzusetzen. So ist es zum Beispiel denkbar, dass 2,6-Naphthalindicarbonsäure in Form ihres Salzes eingesetzt wird. Das Salz, bei dem 2,6-Naphthalindicarbonsäure als vollständig oder teilweise deprotoniertes Anion vorliegt, kann ein beliebig geeignetes Kation aufweisen.

Solche Kationen können beispielsweise ein- oder zweiwertige, vorzugsweise einwertige Metallionen sein. Beispiele hierfür sind insbesondere Natrium- und Kaliumsalze. Ebenso sind Kationen von Ammoniumverbindungen einsetzbar. Hierbei sind insbesondere Ammonium selbst sowie Alkylammoniumkationen zu nennen.

Die Aluminiumverbindung kann durch anodische Oxidation von metallischem Aluminium erzeugt werden. In einem solchen Fall wird das erfindungsgemäße poröse metallorganische Gerüstmaterial auf zumindest teilweise elektrochemischem Wege hergestellt. Verfahren zur elektrochemischen Herstellung von porösen metallorganischen Gerüstmaterialien sind in WO-A 2005/049892 beschrieben. Auch die Aluminiumverbindung für das erfindungsgemäße poröse metallorganische Gerüstmaterial kann auf diesem Wege hergestellt werden.

Bei der elektrochemischen Herstellung des erfindungsgemäßen porösen metallorganischen Gerüstmaterials ist bevorzugt, dass die kathodische Wiederabscheidung des Aluminiumions durch mindestens eine der folgenden Maßnahmen zumindest teilweise verhindert wird:
(i) Verwendung eines Elektrolyten, der die kathodische Bildung von Wasserstoff begünstigt;
(ii) Zusatz mindestens einer Verbindung, die zu einer kathodischen Depolarisation führt;
(iii) Einsatz einer Kathode mit einer geeigneten Wasserstoffüberspannung.

Das Verfahren kann in einer ungeteilten Elektrolysezelle durchgeführt werden. Speziell geeignete Zellen sind Spaltzellen oder Plattenstapelzellen. Diese können bipolar geschaltet sein. Als Reaktionsmedium eignet sich beispielsweise Methanol, Ethanol, Dimethylformamid, Diethylformamid oder ein Gemisch aus zwei oder mehr dieser Lösemittel.

In dem Reaktionsgemisch kann/können weiterhin ein Leitsalz oder mehrere Leitsalze vorhanden sein. Hierbei kann das Leitsalz als Kationkomponente ein quartäres Ammonium und als Anionkomponente ein Alkoxysulfat aufweisen. Der Gesamtfeststoffgehalt sollte im Bereich von größer oder gleich 0,5 Gew.-% liegen.

Die Umsetzung in dem erfindungsgemäßen Verfahren zur Herstellung des erfindungsgemäßen metallorganischen Gerüstmaterials kann auch auf klassischem Wege erfolgen. Hierbei ist die Aluminiumverbindung typischerweise ein Aluminiumsalz.

Das Aluminiumsalz kann in Form eines Alkoholats, Acetonats, Halegonids, Sulfits, als Salz einer organischen oder anorganischen, Sauerstoff enthaltenden Säure oder einer Mischung davon vorliegen.

Ein Alkoholat ist beispielsweise ein Methanolat, Ethanolat, n-Propanolat, i-Propanolat, n-Butanolat, i-Butanolat, t-Butanolat oder Phenolat.

Ein Acetonat ist beispielsweise Acetylacetonat.

Ein Halogenid ist beispielsweise Chlorid, Bromid oder lodid.

Eine organische, Sauerstoff enthaltende Säure ist beispielsweise Ameisensäure, Essigsäure, Propionsäure oder andere Alkylmonocarbonsäuren.

Eine anorganische, Sauerstoff enthaltende Säure ist beispielsweise Schwefelsäure, schweflige Säure, Phosphorsäure oder Salpetersäure.

Weiter bevorzugte Aluminiumverbindungen sind anorganische Aluminiumsalze wie Aluminiumchlorid, Aluminiumbromid, Aluminiumhydrogensulfat, Aluminiumdihydrogenphosphat, Aluminiummonohydrogenphosphat, Aluminiumphosphat, Aluminiumnitrat.

Die Aluminiumverbindung kann gegebenenfalls Hydratwasser aufweisen. Bevorzugt sind als Aluminiumverbindung die Hydrate des Chlorids, Nitrats sowie Sulfats.

Die Umsetzung in dem erfindungsgemäßen Verfahren zur Herstellung des erfindungsgemäßen porösen metallorganischen Gerüstmaterials erfolgt zumindest in Gegenwart eines organischen Lösemittels. Hierbei können Solvothermalbedingungen eingesetzt werden. Unter dem Begriff "thermal" ist im Rahmen der vorliegenden Erfindung ein Herstellverfahren zu verstehen, bei dem die Umsetzung zum erfindungsgemäßen porösen metallorganischen Gerüstmaterial in einem Druckbehälter derart durchgeführt wird, dass dieser während der Umsetzung verschlossen ist und erhöhte Temperatur angelegt wird, so dass aufgrund des Dampfdruckes von vorhandenem Lösemittel sich ein Druck innerhalb des Reaktionsmediums im Druckbehälter aufbaut.

Vorzugsweise erfolgt die Umsetzung nicht in Wasser enthaltendem Medium und ebenso nicht unter Solvothermalbedingungen.

Die Umsetzung in dem erfindungsgemäßen Verfahren erfolgt demzufolge vorzugsweise in Gegenwart eines nicht-wässrigen Lösemittels.

Die Umsetzung erfolgt vorzugsweise bei einem Druck von höchstens 2 bar (absolut). Vorzugsweise beträgt der Druck jedoch höchstens 1230 mbar (absolut). Insbesondere bevorzugt findet die Umsetzung bei Atmosphärendruck statt. Hierbei kann es jedoch apparativ bedingt zu leichten Über- oder Unterdrücken kommen. Daher ist im Rahmen der vorliegenden Erfindung unter dem Begriff "Atmosphärendruck" derjenige Druckbereich zu verstehen, der sich aus dem tatsächlichen vorliegenden Atmosphärendruck ± 150 mbar ergibt.

Die Umsetzung findet in einem Temperaturbereich von 100 °C bis 200 °C statt. Vorzugsweise liegt die Temperatur im Bereich von 110 °C bis 170 °C. Weiterhin bevorzugt liegt die Temperatur in einem Bereich von 120 °C bis 150 °C.

Das Reaktionsgemisch kann weiterhin eine Base aufweisen. Dies dient insbesondere dazu, dass bei Einsatz der Carbonsäure als mindestens zweizähniger organischer Verbindung diese leicht löslich ist. Durch die Verwendung eines organischen Lösemittels ist es häufig nicht erforderlich, eine solche Base einzusetzen. Nichts desto trotz kann das Lösemittel für das erfindungsgemäße Verfahren derart gewählt werden, dass dieses als solches basisch reagiert, was jedoch nicht zwingend für die Durchführung des erfindungsgemäßen Verfahrens sein muss.

Ebenso kann eine Base eingesetzt werden. Bevorzugt ist jedoch, dass keine zusätzliche Base eingesetzt wird.

Es ist weiterhin vorteilhaft, dass die Umsetzung unter Rühren stattfinden kann, was auch bei einem Scale-up vorteilhaft ist.

Das (nicht-wässrige) organische Lösemittel ist vorzugsweise ein C₁₋₆-Alkanol, Dimethylsulfoxid (DMSO), N,N-Dimethylformamid (DMF), N,N-Diethylformamid (DEF), N,N-Dimethylacetamid (DMAc), Acetonitril, Toluol, Dioxan, Benzol, Chlorbenzol, Methylethylketon (MEK), Pyridin, Tetrahydrofuran (THF), Essigsäureethylester, gegebenenfalls halogeniertes C₁₋₂₀₀-Alkan, Sulfolan, Glykol, N-Methylpyrrolidon (NMP), gamma-Butyrolacton, alicyclische Alkohole wie Cyclohexanol, Ketone, wie Aceton oder Acetylaceton, Cycloketone, wie Cyclohexanon, Sulfolen oder Mischungen davon.

Ein C₁₋₆-Alkanol bezeichnet einen Alkohol mit 1 bis 6 C-Atomen. Beispiele hierfür sind Methanol, Ethanol, n-Propanol, i-Propanol, n-Butanol, i-Butanol, t-Butanol, Pentanol, Hexanol sowie Gemische davon.

Ein gegebenenfalls halogeniertes C₁₋₂₀₀-Alkan bezeichnet ein Alkan mit 1 bis 200 C-Atomen, wobei ein oder mehrere bis hin zu allen Wasserstoffatomen durch Halogen, vorzugsweise Chlor oder Fluor, insbesondere Chlor, ersetzt sein kann bzw. können. Beispiele hierfür sind Chloroform, Dichlormethan, Tetrachlormethan, Dichlorethan, Hexan, Heptan, Oktan sowie Gemische davon.

Bevorzugte Lösemittel sind DMF, DEF, DMAc und NMP. Besonders bevorzugt ist DMF.

Der Begriff "nicht-wässrig" bezieht sich vorzugsweise auf ein Lösemittel, das einen Höchstwassergehalt von 10 Gew.-%, mehr bevorzugt 5 Gew.-%, weiterhin mehr bevorzugt 1 Gew.-%, weiterhin bevorzugt 0,1 Gew.-%, besonders bevorzugt 0,01 Gew.-% bezogen auf das Gesamtgewicht des Lösemittels nicht überschreitet.

Vorzugsweise beträgt der Höchstwassergehalt während der Umsetzung 10 Gew.-%, mehr bevorzugt 5 Gew.-% und weiterhin mehr bevorzugt 1 Gew.-%.

Der Begriff "Lösemittel" betrifft reine Lösemittel sowie Gemische von unterschiedlichen Lösemitteln.

Weiterhin bevorzugt schließt sich an den Verfahrensschritt der Umsetzung der mindestens einen Metallverbindung mit der mindestens einen mindestens zweizähnigen organischen Verbindung ein Calcinierungsschritt an. Die hierbei eingestellte Temperatur beträgt typischerweise mehr als 250 °C, bevorzugt 300 bis 400 °C.

Aufgrund des Calcinierungsschrittes kann die in den Poren befindliche mindestens zweizähnige organische Verbindung entfernt werden.

Ergänzend oder alternativ hierzu kann die Entfernung der mindestens zweizähnigen organischen Verbindung (Ligand) aus den Poren des porösen metallorganischen Gerüstmaterials durch die Behandlung des gebildeten Gerüstmaterials mit einem nicht-wässrigen Lösemittel erfolgen. Hierbei wird in einer Art "Extraktionsverfahren" der Ligand entfernt und gegebenenfalls im Gerüstmaterial durch ein Lösemittelmolekül ersetzt.

Die Behandlung erfolgt vorzugsweise mindestens 30 Minuten und kann typischerweise bis zu 7 Tagen durchgeführt werden. Dies kann bei Raumtemperatur oder erhöhter Temperatur geschehen. Vorzugsweise erfolgt dies unter erhöhter Temperatur, beispielsweise bei mindestens 40 °C, bevorzugt 60 °C. Weiterhin bevorzugt erfolgt die Extraktion bei der Siedetemperatur des eingesetzten Lösemittels statt (unter Rückfluss).

Die Behandlung kann in einem einfachen Kessel durch Aufschlämmen und Rühren des Gerüstmaterials erfolgen. Es können auch Extraktionsapparaturen wie Soxhlet-Apparaturen, insbesondere technische Extraktionsapparaturen, eingesetzt werden.

Als geeignete Lösemittel können die oben genannten verwendet werden, also beispielsweise C₁₋₆-Alkanol, Dimethylsulfoxid (DMSO), N,N-Dimethylformamid (DMF), N,N-Diethylformamid (DEF), N,N-Dimethylacetamid (DMAc), Acetonitril, Toluol, Dioxan, Benzol, Chlorbenzol, Methylethylketon (MEK), Pyridin, Tetrahydrofuran (THF), Essigsäureethylester, gegebenenfalls halogeniertes C₁₋₂₀₀-Alkan, Sulfolan, Glykol, N-Methylpyrrolidon (NMP), gamma-Butyrolacton, alicyclische Alkohole wie Cyclohexanol, Ketone, wie Aceton oder Acetylaceton, Cycloketone, wie Cyclohexanon oder Mischungen davon.

Bevorzugt sind Methanol, Ethanol, Propanol, Aceton, MEK und Mischungen davon.

Ein ganz besonders bevorzugtes Extraktionslösemittel ist Methanol.

Das verwendete Lösemittel zur Extraktion kann gleich oder verschieden zu demjenigen für die Umsetzung der mindestens einen Metallverbindung mit der mindestens einen mindestens zweizähnigen organischen Verbindung sein. Es ist bei der "Extraktion" nicht unbedingt erforderlich aber bevorzugt, dass das Lösemittel wasserfrei ist.

Nach dem erfindungsgemäßen Verfahren beträgt die Summe der Anteile an der Aluminiumverbindung und der 2,6-Naphthalindicarbonsäure oder einem ihrer Salze (X₁) weniger als 20 Gew.-% bezogen auf das Gesamtgewicht des Reaktiorisgemisches. Vorzugsweise beträgt dieser Anteil weniger als 15 Gew.-%, insbesondere weniger als 12 Gew.-%. Dieser Anteil sollte jedoch größer als 2 Gew.-% sein, bevorzugt größer als 2,7 Gew.-%.

Es ist bevorzugt, dass in dem erfindungsgemäßen Verfahren das molare Verhältnis Aluminiumverbindung zu 2,6-Naphthalindicarbonsäure oder einem ihrer Salze (x₂) im Bereich von 0,3 bis 1,5 liegt. Mehr bevorzugt liegt das Verhältnis im Bereich von 0,4 bis 0,8. Ein weiterer bevorzugter Bereich für das Verhältnis ist der Bereich ab 2, insbesondere ab 2,6; der Wert sollte jedoch 5 nicht übersteigen.

Ein weiterer Gegenstand der vorliegenden Erfindung ist die Verwendung eines erfindungsgemäßen porösen metallorganischen Gerüstmaterials zur Aufnahme mindestens eines Stoffes, zu dessen Speicherung, Abtrennung, kontrollierten Abgabe, chemischen Umsetzung oder als Träger.

Vorzugsweise handelt es sich bei dem mindestens einen Stoff um ein Gas oder Gasgemisch.

Verfahren zur Speicherung mit Hilfe von metallorganischen Gerüstmaterialien im Allgemeinen sind in WO-A 2005/003622, WO-A 2003/064030, WO-A 2005/049484, WO-A 2006/089908 sowie DE-A 10 2005 012 087 beschrieben. Die dort beschriebenen Verfahren können auch für das erfindungsgemäße metallorganische Gerüstmaterial eingesetzt werden.

Verfahren zur Trennung beziehungsweise Reinigung mit Hilfe von metallorganischen Gerüstmaterialien im Allgemeinen sind in der EP-A 1 674 555, DE-A 10 2005 000938 und in der deutschen Patentanmeldung mit der Anmeldenummer DE-A 10 2005 022 844 beschrieben. Die dort beschriebenen Verfahren können auch für das erfindungsgemäße metallorganische Gerüstmaterial eingesetzt werden.

Sofern das erfindungsgemäße poröse metallorganische Gerüstmaterial zur Speicherung eingesetzt wird, erfolgt dies vorzugsweise in einem Temperaturbereich von -200 °C bis +80 °C. Mehr bevorzugt ist ein Temperaturbereich von -40 °C bis +80 °C.

Im Rahmen der vorliegenden Erfindung werden vereinfachend die Begriffe "Gas" und "Flüssigkeit" verwendet, wobei hier jedoch ebenso Gasgemische sowie Flüssigkeitsgemische beziehungsweise flüssige Lösungen unter dem Begriff "Gas" beziehungsweise "Flüssigkeit" zu verstehen sind.

Bevorzugte Gase sind Wasserstoff, Erdgas, Stadtgas, Kohlenwasserstoffe, insbesondere Methan, Ethan, Ethen, Acetylen, Propan, n-Butan sowie i-Butan, Kohlenmonoxid, Kohlendioxid, Stickoxide, Sauerstoff, Schwefeloxide, Halogene, halogenide Kohlenwasserstoffe, NF₃, SF₆, Ammoniak, Borane, Phosphane, Schwefelwasserstoff, Amine, Formaldehyd, Edelgase, insbesondere Helium, Neon, Argon, Krypton sowie Xenon.

Besonders bevorzugt handelt es sich bei dem Gas um Kohlendioxid, das aus einem Kohlendioxid enthaltenden Gasgemisch abgetrennt wird. Bevorzugt weist dabei das Gasgemisch neben Kohlendioxid wenigstens H₂, CH₄ oder Kohlenmonoxid auf. Insbesondere weist dabei das Gasgemisch neben Kohlendioxid Kohlenmonoxid auf. Ganz besonders bevorzugt sind Gemische, die wenigstens 10 und höchsten 45 Vol.-% Kohlendioxid und wenigstens 30 und höchstens 90 Vol.-% Kohlenmonoxid enthalten.

Eine bevorzugte Ausführungsform ist die Druckwechseladsorption mit mehreren parallelen Adsorberreaktoren, wobei die Adsorbensschüttung ganz oder teilweise aus dem erfindungsgemäßen Material besteht. Die Adsorptionsphase findet für die CO₂/CO-Trennung bevorzugt bei einem CO₂-Partialdruck von 0,6 bis 3 bar und Temperatur von wenigstens 20, jedoch höchstens 70°C statt. Zur Desorption des adsorbierten Kohlendioxids wird der Gesamtdruck in dem betreffenden Adsorberreaktor üblicherweise abgesenkt auf Werte zwischen 100 mbar und 1 bar.

Weiterhin bevorzugt ist die Verwendung des erfindungsgemäßen Gerüstmaterials zum Speichern eines Gases bei einem Mindestdruck von 100 bar (absolut). Mehr bevorzugt beträgt der Mindestdruck 200 bar (absolut), insbesondere 300 bar (absolut). Hierbei handelt es sich besonders bevorzugt bei dem Gas um Wasserstoff oder Methan.

Bei dem mindestens einen Stoff kann es sich jedoch auch um eine Flüssigkeit handeln. Beispiele für eine solche Flüssigkeit sind Desinfektionsmittel, anorganische oder organische Lösemittel, Treibstoffe - insbesondere Benzin oder Diesel -, Hydraulik-, Kühler-, Bremsflüssigkeit oder ein Öl, insbesondere Maschinenöl. Weiterhin kann es sich bei der Flüssigkeit um halogenierte aliphatische oder aromatische, cyclische oder acyclische Kohlenwasserstoffe oder Mischungen davon handeln. Insbesondere kann die Flüssigkeit Aceton, Acetonitril, Anilin, Anisol, Benzol, Benzonitril, Brombenzol, Butanol, tert.-Butanol, Chinolin, Chlorbenzol, Chloroform, Cyclohexan, Diethylenglykol, Diethylether, Dimethylacetamid, Dimethylformamid, Dimethylsulfoxid, Dioxan, Eisessig, Essigsäureanhydrid, Essigsäureethylester, Ethanol, Ethylencarbonat, Ethylendichlorid, Ethylenglykol, Ethylenglykoldimethylether, Formamid, Hexan, Isopropanol, Methanol, Methoxypropanol, 3-Methyl-1-butanol, Methylenchlorid, Methylethylketon, N-Methylformamid, N-Methylpyrrolidon, Nitrobenzol, Nitromethan, Piperidin, Propanol, Propylencarbonat, Pyrridin, Schwefelkohlenstoff, Sulfolan, Tetrachlorethen, Tetrachlorkohlenstoff, Tetrahydrofuran, Toluol, 1,1,1-Trichlorethan, Trichlorethylen, Triethylamin, Triethylenglykol, Triglyme, Wasser oder Mischungen hiervon handeln.

Weiterhin kann der mindestens eine Stoff ein Geruchsstoff sein.

Vorzugsweise handelt es sich bei dem Geruchsstoff um eine flüchtige organische oder anorganische Verbindung, die mindestens eines der Elemente Stickstoff, Phosphor, Sauerstoff, Schwefel, Fluor, Chlor, Brom oder lod enthält oder ein ungesättigter oder aromatischer Kohlenwasserstoff oder ein gesättigter oder ungesättigter Aldehyd oder ein Keton ist. Mehr bevorzugte Elemente sind Stickstoff, Sauerstoff, Phosphor, Schwefel, Chlor, Brom; insbesondere bevorzugt sind Stickstoff, Sauerstoff, Phosphor und Schwefel.

Insbesondere handelt es sich bei dem Geruchsstoff um Ammoniak, Schwefelwasserstoff, Schwefeloxide, Stickoxide, Ozon, cyclische oder acyclische Amine, Thiole, Thioether sowie Aldehyde, Ketone, Ester, Ether, Säuren oder Alkohole. Besonders bevorzugt sind Ammoniak, Schwefelwasserstoff, organische Säuren (bevorzugt Essigsäure, Propionsäure, Buttersäure, Isobuttersäure, Valeriansäure, Isovaleriansäure, Capronsäure, Heptylsäure, Laurinsäure, Pelargonsäure) sowie cyclische oder acyclische Kohlenwasserstoffe, die Stickstoff oder Schwefel enthalten sowie gesättigte oder ungesättigte Aldehyde, wie Hexanal, Heptanal, Oktanal, Nonanal, Decanal, Octenal oder Nonenal und insbesondere flüchtige Aldehyde wie Butyraldehyd, Propionaldehyd, Acetaldehyd und Formaldehyd und weiterhin Treibstoffe wie Benzin, Diesel (Inhaltsstoffe).

Bei den Geruchsstoffen kann es sich auch um Riechstoffe, die beispielsweise zur Herstellung von Parfümen verwendet werden, handeln. Beispielhaft seien als Riechstoffe oder Öle, die solche Riechstoffe freisetzen zu nennen: ätherische Öle, Basilikumöl, Geranienöl, Minzöl, Canangabaumöl, Kardamomöl, Lavendelöl, Pfefferminzöl, Muskatöl, Kamillenöl, Eukalyptusöl, Rosmarinöl, Zitronenöl, Limettenöl, Orangenöl, Bergamottenöl, Muskateller Salbeiöl, Korianderöl, Zypressenöl, 1,1-Dimethoxy-2-pherylethan, 2,4-Dimethyl-4-phenyltetrahydrofuran, Dimethyltetrahydrobenzaldehyd, 2,6-Dimethyl-7-octen-2-ol, 1,2-Diethoxy-3,7-dimethyl-2,6-octadien, Phenylacetaldehyd, Rosenoxid, Ethyl-2-methylpentanoat, 1-(2,6,6-Trimethyl-1,3-cyclohexadien-1-yl)-2-buten-1-on, Ethylvanillin, 2,6-Dimethyl-2-octenol, 3,7-Dimethyl-2-octenol, tert-Butylcyclohexylacetat, Anisylacetate, Allylcyclohexyloxyacetat, Ethyllinalool, Eugenol, Cumarin, Ethylacetacetat, 4-Phenyl-2,4,6-trimethyl-1,3-dioxan, 4-Methylen-3,5,6,6-tetramethyl-2-heptanon, Ethyltetrahydrosafranat, Geranylnitril, cis-3-Hexen-1-ol, cis-3-Hexenylacetat, cis-3-Hexenylmethylcarbonate, 2,6-Dimethyl-5-hepten-1-al, 4-(Tricyclo[5.2.1.0]decylidene)-8-butanal, 5-(2,2,3-Trimethyl-3-cyclopentenyl)-3-methylpentan-2-ol, p-tert-Butylalpha-methylhydrozimtaldehyd, Ethyl[5.2.1.0]tricyclodecancarboxylat, Geraniol, Citronellol, Citral, Linalool, Linalylacetat, Jonone, Phenylethanol oder Mischungen hiervon.

Im Rahmen der vorliegenden Erfindung weist ein flüchtiger Geruchsstoff vorzugsweise einen Siedepunkt oder Siedepunktsbereich von weniger als 300 °C auf. Mehr bevorzugt ist der Geruchsstoff eine leicht flüchtige Verbindung oder Gemisch. Insbesondere bevorzugt weist der Geruchsstoff einen Siedepunkt oder Siedebereich von weniger als 250 °C, mehr bevorzugt weniger als 230 °C, insbesondere bevorzugt weniger als 200 °C auf.

Bevorzugt sind ebenfalls Geruchsstoffe, die eine hohe Flüchtigkeit aufweisen. Als Maß für die Flüchtigkeit kann der Dampfdruck herangezogen werden. Im Rahmen der vorliegenden Erfindung weist ein flüchtiger Geruchsstoff vorzugsweise einen Dampfdruck von mehr als 0,001 kPa (20 °C) auf. Mehr bevorzugt ist der Geruchsstoff eine leicht flüchtige Verbindung oder Gemisch. Insbesondere bevorzugt weist der Geruchsstoff einen Dampfdruck von mehr als 0,01 kPa (20 °C) auf, mehr bevorzugt einen Dampfdruck von mehr als 0,05 kPa (20 °C) auf. Besonders bevorzugt weisen die Geruchsstoffe einen Dampfdruck von mehr als 0,1 kPa (20 °C) auf.

Beispiele, bei der eine chemische Umsetzung in Gegenwart des erfindungsgemäßen metallorganischen Gerüstmaterials stattfinden kann, stellen die Alkoxylierung von Monoolen sowie Polyolen dar. Die Durchführung solcher Alkoxylierungen sind WO-A 03/035717 sowie WO-A 2005/03069 beschrieben. Ebenso kann das erfindungsgemäße poröse metallorganische Gerüstmaterial zur Epoxidierung sowie Herstellung von Polyalkylencarbonaten und Wasserstoffperoxid eingesetzt werden. Solche Reaktionen sind in WO-A 03/101975, WO-A 2004/037895 sowie US-A 2004/081611 beschrieben.

Besonders bevorzugt sind katalytische Umsetzungen.

Darüber hinaus kann das erfindungsgemäße metallorganische Gerüstmaterial als Träger, insbesondere als Träger eines Katalysators, dienen.

### Beispiele

### Beispiel 1: Herstellung eines erfindungsgemäßen AI-2,6-Naphthalindicarbonsäure-metallorganischen Gerüstmaterials (AI-NDC)

5 g AlCl₃*6H₂O und 6,72 g 2,6-Naphthalindicarbonsäure (molares Verhältnis: AI:Linkerverhältnis, x2, = 0,66) werden in einem Glaskolben in 300 ml DMF suspendiert (Summe der Eduktanteile zu Gesamtgewicht des Reaktionsgemisches in Gew.-%, x1, 3,9) und 17 h unter Rückfluss (130°C) gerührt. Der entstandene Feststoff wird abfiltriert und mit 3 x 50 ml DMF und 4 x 50 ml Methanol gewaschen. Anschließend wird die Substanz im Vakuumtrockenschrank bei 110°C 20 h getrocknet. Es werden 6,89 g eines weißen Vorprodukts erhalten. Die N₂-Oberfläche wird mit 269 m²/g (Langmuir) bestimmt.

1,71 g des Vorprodukts werden in einer Extraktiohsapparatur 4 Tage mit siedendem Methanol extrahiert. Anschließend wird das Produkt (1,09 g) 16 h bei 150°C im Vakuumtrockenschrank getrocknet. Dieses Produkt weist eine spezifische Oberfläche nach Langmuir von 1988 m²/g auf. Das XRD ist in Fig. 1 dargestellt. Hierbei beschreiben in dem Diffraktogramm wie auch in den übrigen Diffraktogrammen I die Intensität (Lin (Counts)) und 2Θ die 2-Theta-Skala.

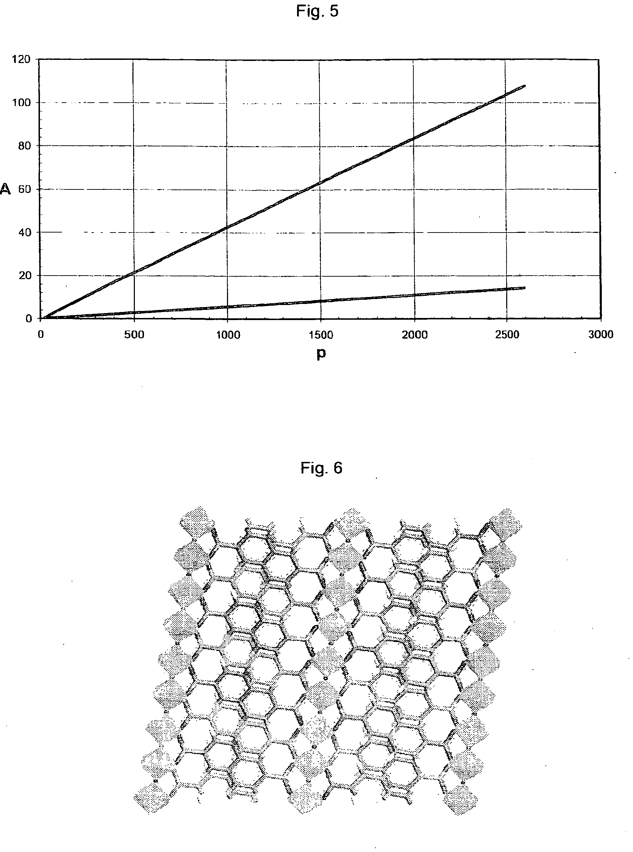

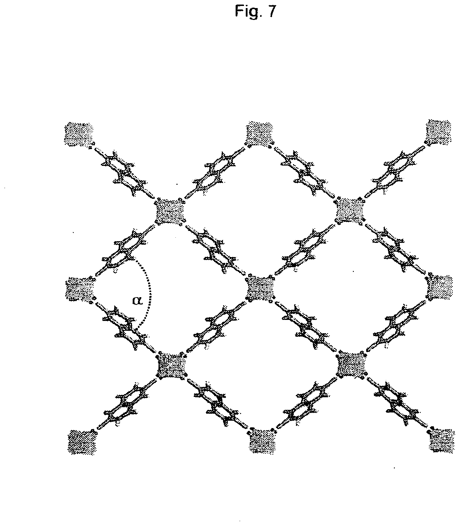

Ein Strukturvorschlag ist in den Fig. 6 (Ebene a, c) bzw. 7 (Ebene a, b) dargestellt. Es handelt sich um eine orthorhomische Struktur. Der Öffnungswinkel α beträgt bei dieser Struktur 82,5° (im Vergleich zu ca. 35° für MIL-69).

Ausgehend von der im Stand der Technik bekannten Struktur wird eine Abtastung des b-Vektors der Al-NDC-Zelle von 14 bis 18 Ä in 0,5 Ä Schritten durchgeführt. Alle anderen Zellparameter (a, b, c) und die molekulare Struktur werden im Hinblick auf jeden b-Wert innerhalb der C2c Raumgruppe optimiert, wobei das "OFF Kraftfeld", wie es in der Cerius Programmfolge implementiert wurde, verwandelt wird. Die XRD-Muster an jedem der wiederoptimierten Abtastpunkte werden mit Hilfe des "Reflex powder diffraction" Moduls des Softwarepakets Materials Studio V2.1 (Acceloys Inc., San Diego, US) erzeugt.

Die so erhaltenen XRD-Muster werden durch Überlagerung mit der experimentell erhaltenen XRD-Struktur verglichen und der geeignete Bereich für die x-Achse ausgewählt. Die y-Achse wird dann wieder skaliert, so dass die Intensitäten des stärksten Reflexes übereinstimmt.

Auf dieser Basis wird initial auf visuellem Weg gefunden, dass die Struktur mit "b" = 18 Ä am besten zu den experimentellen Daten korreliert. Trotzdem stimmen alle Strukturen mit Werten im Bereich von 18 bis 16,5 Ä aufgrund des relativ breiten Reflexes gut mit dem experimentellen überein.

Dies erlaubt einen gewissen Spielraum für die Wahl einer geeigneten b Länge. Wenn beide Positionen der Reflexe und Intensitäten berücksichtigt werden, ergibt sich, dass eine Struktur mit einem b Vektor von 16,5 Ä Länge am besten mit dem experimentellen XRD übereinstimmt. Strukturen mit geringeren als 16,5 Ä Werten für b verschieben in Richtung auf zu hohe Theta-Werte, wobei die Intensität und Positionen nicht mehr übereinstimmen. Alle diese Strukturen können als mögliche Lösungen außer Acht gelassen werden.

Die Strukturen, welche mit den "erlaubten" b-Längenbereichen erhalten werden, entsprechen den folgenden internen Al-Al-Al Winkeln (verglichen mit den experimentellen von MIL-69* und den ht**- beziehungsweise as***-Strukturen von Aluminiumterephthalat):

| b (Å) | Winkel (AI-AI-AI) |
|---|---|
| 18,0 | 90 |
| 17,5 | 87 |
| 17.0 | 84 |
| 16,5 | 81 |
| 7,53 | 35* |
| | 71** |
| | 75*** |

Dieser Vergleich zeigt, dass sich sowohl die Zelldimension als auch die interne Struktur der erfindungsgemäßen Gerüstmaterialien von denen aus dem Stand der Technik unterscheiden. In der neuen Struktur sind offenere Kanalstrukturen zu finden im Vergleich zu denen aus dem Stand der Technik.

Basierend auf der Übereinstimmung der Positionen der Reflexe und Intensität zwischen experimentellen XRD-Mustern und Simultanionsmustern wird als sehr wahrscheinliche Struktur diejenige mit den folgenden Koordinaten vorgeschlagen:

| Raumgruppe | C2c | | |
|---|---|---|---|
| **a** | 19.38570 | **alpha** | 90.0 |
| **b** | 16.500000 | **beta** | 90.0 |
| **c** | 6.11620 | **gamma** | 101.6 |

| Fraktionale Koordinaten | | | |
|---|---|---|---|
| **Atom** | **a** | **b** | **c** |
| O | 0.06478 | -0.92638 | 0.60817 |
| O | 0.06785 | -0.93013 | 0.97876 |
| C | 0.14628 | -0.84122 | 0.84478 |
| C | 0.19227 | -0.83455 | 0.05343 |
| C | 0.29301 | 0.77063 | 0.29415 |
| C | 0.08982 | -0.90275 | 0.80853 |
| C | 0.24629 | -0.77654 | 0.08763 |
| C | 0.34566 | -0.71153 | 0.32867 |
| H | 0.31342 | -0.37510 | 0.31175 |
| H | 1.28863 | -0.18852 | -1.07063 |
| H | 2.38077 | -0.29217 | -1.00960 |
| Al | 0.00000 | 0.00000 | 0.00000 |
| O | 0.00000 | -0.03397 | 0.75000 |

Dies ist die theoretisch simulierte Struktur, die am besten mit dem experimentell bestimmten XRD Muster übereinstimmt und auch ein Minimum bei der abgetasteten potentiellen Energieoberfläche aufweist. Jedoch wird auch eine weitere Struktur mit einem Minimum nahe dieser Struktur (b = 18) gefunden, wobei deren Parameter trotz der geringeren Übereinstimmung nachfolgend aufgeführt sind:

| Raumgruppe | C2c | | |
|---|---|---|---|
| **a** | 17.99800 | **alpha** | 90.0 |
| **b** | 18.00000 | **beta** | 90.0 |
| **C** | 6.11610 | **gamma** | 101.7 |

| Fraktionale Koordinaten | | | |
|---|---|---|---|
| **Atom** | **a** | **b** | **c** |
| O | 0.06942 | -0.93200 | 0.60934 |
| O | 0.07288 | -0.93525 | 0.98028 |
| C | 0.15102 | -0.84750 | 0.84396 |
| C | 0.19865 | -0.83551 | 0.05439 |
| C | 0.29732 | 0.76340 | 0.29709 |
| C | 0.09525 | -0.90894 | 0.80963 |
| C | 0.24903 | -0.77499 | 0.08855 |
| C | 0.34605 | -0.70216 | 0.33147 |
| H | 0.30339 | -0.37327 | 0.30879 |
| H | 1.29741 | -0.19830 | -1.06623 |
| H | 2.38290 | -0.30529 | -1.00569 |
| Al | 0.00000 | 0.00000 | 0.00000 |
| O | 0.00000 | -0.03093 | 0.75000 |

### Vergleichsbeispiel 1: Herstellung eines Al-NDC-MOFs ("MIL-69")

15,31 g Al(NO₃)₃*9H₂O und 15,04 g 2,6-Naphthalindicarbonsäure (x2 = 0,58) werden in einem Glaskolben in 102 g DMF suspendiert (x1 = 29) und in einem Berghoff-Autoklaven ("Teflon-Liner") 24 h bei 170°C gehalten. Der entstandene Feststoff wird abfiltriert und mit 2x50 ml DMF und 3x100 ml Methanol gewaschen. Anschließend wird die Substanz im Vakuumtrockenschrank bei 200°C 5 h getrocknet. Danach wird das Vorprodukt in einem großen Überschuss Methanol mehrere Stunden unter Rückfluss extrahiert, abfiltriert und mehrmals mit Methanol nachgewaschen und wiederum 5 h bei 200°C im Vakuumtrockenschrank getrocknet. Es wird ein weißes Produkt mit einer N₂-Oberfläche von nur noch 238 m²/g (Langmuir) gewonnen. Das XRD ist in Fig. 2 dargestellt. Anhand der Lage der Reflexe kann das Produkt als die aus der Literatur bekannte "MIL-69"-Phase identifiziert werden.

### Vergleichsbeispiel 2: Nicht erfindungsgemäße Herstellung eines Gemisches aus erfindungsgemäßem und bekanntem Gerüstmaterial AI-NDC

59,2 g AlCl₃*6H₂O und 90,3 g 2,6-Naphthalindicarbonsäure (x2 = 0,59) werden in einem Glaskolben in 645 ml DMF suspendiert (x1 = 23) und 17 h unter Rückfluss (130°C) gerührt. Der entstandene Feststoff wird abfiltriert und mit 2x150 ml DMF und 4x150 ml Methanol gewaschen. Anschließend wird die Substanz im Vakuumtrockenschrank bei 110°C 16 h getrocknet. Danach wird das Vorprodukt in einem großen Überschuss Methanol mehrere Stunden unter Rückfluss extrahiert, abfiltriert und mehrmals mit Methanol nachgewaschen und wiederum 16 h bei 110°C im Vakuumtrockenschrank getrocknet. Es wird ein weißes Produkt mit einer N₂-Oberfläche von nur noch 510 m²/g (Langmuir) gewonnen. Das XRD ist in Fig. 3 dargestellt. Es handelt sich vermutlich um eine Mischung aus der erfindungsgemäßen Struktur und MIL-69.

### Beispiel 2: Wasserstoff-Isothermen bei 77 K (Speicherung von Wasserstoff)

In Fig. 4 ist der Vergleich der H₂-Aufnahme A (ml/g) als Funktion des relativen Druckes p/p0 dargestellt. Gemessen wird an einem kommerziell erhältlichen Gerät der Fa. Quantachrome mit der Bezeichnung Autosorb-1. Die Messtemperatur liegt bei 77,4 K. Die Proben werden vor der Messung jeweils 4 h bei Raumtemperatur und anschließend weitere 4 h bei 200°C im Vakuum vorbehandelt.

Die obere Kurve in Fig. 4 zeigt die H₂-Aufnahme an dem Gerüstmaterial aus Beispiel 1. Die untere Kurve bezieht sich auf Al-Terephthalat, das gemäß Beispiel 1 von DE-A 10 2005 039 623 hergestellt und das nach der Fällung analog dem obigen Beispiel 1 mit Methanol extrahiert statt calciniert wurde.

### Beispiel 3: Herstellung eines erfindungsgemäßen Gerüstmaterials

10 g AlCl₃*6H₂O und 13,44 g 2,6-Naphthalindicarbonsäure (x2 = 0,66) werden in einem Glaskolben in 600 ml DMF suspendiert (x1 = 3,9) und 17 h unter Rückfluss (130°C) gerührt. Der entstandene Feststoff wird abfiltriert und mehrmals mit Aceton gewaschen. Danach wird das Vorprodukt in einem großen Überschuss Methanol mehrere Stunden unter Rückfluss extrahiert, abfiltriert und mehrmals mit Methanol nachgewaschen. Anschließend wird die Substanz im Vakuumtrockenschrank bei 110°C 16 h getrocknet. Es werden 11,5 g eines weißen Vorprodukts erhalten. Dieses Produkt weist eine N₂-Oberfläche von 2085 m²/g (Langmuir) auf. Das XRD ist ähnlich zu dem aus Beispiel 1.

### Beispiel 4: Eignung für die CO₂ -Trennungen

Es werden bei 42°C Reinstoffisothermen von CO₂ und CO an dem Material aus Bsp. 3 aufgenommen (Fig. 5).

In Fig. 5 zeigt die obere Kurve die adsorbierte Menge A (mg/g) von Kohlendioxid bei 42 °C und die untere Kurve von Kohlenmonoxid jeweils als Funktion des absoluten Drucks (mbar). Anhand der deutlich unterschiedlichen Kurvenlage ist das erfindungsgemäße Gerüstmaterial prinzipiell für die Trennung von CO₂ aus Gasgemischen geeignet, die weiterhin Kohlenmonoxid enthalten.

### Beispiel 5: Herstellung des erfindungsgemäßen Gerüstmaterials

7,5 g AlCl₃*6H₂O und 10 g 2,6-Naphthalindicarbonsäure (x2 = 0,66) werden in einem Glaskolben in 300 ml DMF suspendiert (x1 = 5,8) und 17 h unter Rückfluss (130°C) gerührt. Der entstandene Feststoff wird abfiltriert und mit 2x50 ml DMF und 4x50 ml Methanol gewaschen. Anschließend wird die Substanz im Vakuumtrockenschrank bei 110°C 16 h getrocknet. Danach wird das Vorprodukt in einem großen Überschuss Methanol mehrere Stunden unter Rückfluss extrahiert, abfiltriert und mehrmals mit Methanol nachgewaschen und wiederum 16 h bei 110°C im Vakuumtrockenschrank getrocknet. Es wird ein weißes Produkt mit einer N₂-Oberfläche von 1866 m²/g (Langmuir) erhalten. Das XRD ist ähnlich zu dem aus Beispiel 1.

### Beispiel 6: Herstellung des erfindungsgemäßen Gerüstmaterials

15 g AlCl₃*6H₂O und 5 g 2,6-Naphthalindicarbonsäure (x2 = 2,66) werden in einem Glaskolben in 300 ml DMF suspendiert (x1 = 6,6) und 17 h unter Rückfluss (130°C) gerührt. Der entstandene Feststoff wird abfiltriert und mit 2x50 ml DMF und 4x50 ml Methanol gewaschen. Anschließend wird die Substanz im Vakuumtrockenschrank bei 110°C 16 h getrocknet. Danach wird das Vorprodukt in einem großen Überschuss Methanol mehrere Stunden unter Rückfluss extrahiert, abfiltriert und mehrmals mit Methanol nachgewaschen und wiederum 16 h bei 110°C im Vakuumtrockenschrank getrocknet. Es wird ein weißes Produkt mit einer N₂-Oberfläche von 1517 m²/g (Langmuir) erhalten. Das XRD ist ähnlich zu dem aus Beispiel 1.

### Beispiel 7: Herstellung des erfindungsgemäßen Gerüstmaterials

1,25 g AlCl₃*6H₂O und 1,64 g 2,6-Naphthalindicarbonsäure (x2 = 0,66) werden in einem Glaskolben in 142 g DMF suspendiert (x1 = 2) und in einem Berghoff-Autoklaven ("Teflon-Liner") 24 h bei 170°C gehalten. Der entstandene Feststoff wird abfiltriert und mit 2x50 ml DMF und 4x50 ml Methanol gewaschen. Anschließend wird die Substanz im Vakuumtrockenschrank bei 110°C 16 h getrocknet. Danach wird das Vorprodukt in einem großen Überschuss Methanol mehrere Stunden unter Rückfluss extrahiert, abfiltriert und mehrmals mit Methanol nachgewaschen und wiederum 16 h bei 110°C im Vakuumtrockenschrank getrocknet. Es wird ein weißes Produkt mit einer N₂-Oberfläche von nur noch 1005 m²/g (Langmuir) gewonnen. Das XRD ist ähnlich zu Fig. 1, jedoch sehr schlecht kristallin.

### Beispiel 8: Herstellung des erfindungsgemäßen Gerüstmaterials

120,72 g AlCl₃*6H₂O und 183,76 g 2,6-Naphthalindicarbonsäure (x2 = 0,59) werden in einem Glaskolben in 2400 ml DMF suspendiert (x1 = 12,7) und unter Rückfluss bei 130°C 17 h gerührt. Der entstandene Feststoff wird abfiltriert und mit 3x400 ml DMF und 3x400 ml Methanol gewaschen. Danach wird das Vorprodukt in 2,51 Methanol 24 Stunden unter Rückfluss extrahiert, abfiltriert und mehrmals mit Methanol nachgewaschen. Anschließend wird die Substanz im Vakuumtrockenschrank bei 100°C 24 h getrocknet. Es wird ein weißes Produkt mit einer N₂-Oberfläche von 1465 m²/g (Langmuir) gewonnen.

### Beispiel 9: Herstellung des erfindungsgemäßen Gerüstmaterials

181,07 g AlCl₃*6H₂O und 275,64 g 2,6-Naphthalindicarbonsäure (x2 = 0,59) werden in einem Glaskolben in 5000 ml DMF suspendiert (x1 = 9,1) und unter Rückfluss bei 130°C 17 h gerührt. Der entstandene Feststoff wird abfiltriert und mit 3x400 ml DMF und 3x400 ml Methanol gewaschen. Danach wird das Vorprodukt in 2,5 I Methanol 24 Stunden unter Rückfluss extrahiert, abfiltriert und mehrmals mit Methanol nachgewaschen. Anschließend wird die Substanz im Vakuumtrockenschrank bei 100°C 24 h getrocknet. Es werden 198 g weißes Produkt mit einer N₂-Oberfläche von 2019 m²/g (Langmuir) gewonnen.

## Patentansprüche

1. Verfahren zur Herstellung eines porösen metallorganischen Gerüstmaterials, enthaltend eine an ein Metallion koordinativ gebundene, zweizähnige organische Verbindung, wobei das Metallion Al^{III} und die zweizähnige organische Verbindung 2,6-Naphthalindicarboxylat ist, **dadurch gekennzeichnet, dass** das Röntgendiffraktogramm (XRD) des Gerüstmaterials im Bereich von 6,5° < 2Θ < 7,5° einen ersten Reflex und im Bereich von 13,8° < 2Θ < 15,0° einen zweiten Reflex aufweist, wobei in Bezug auf die Fläche aller Reflexe im Bereich von 2° < 2Θ < 70° die Fläche des ersten Reflexes am größten und die Fläche des zweiten Reflexes am zweitgrößten ist und wobei die Summe der Flächen von erstem und zweitem Reflex in Bezug auf die Gesamtfläche aller Reflexe im Bereich von 2° < 2Θ < 70° mindestens 50 % ergibt, den Schritt enthaltend
- Umsetzung eines Reaktionsgemisches enthaltend eine Aluminiumverbindung, 2,6-Naphthalindicarbonsäure oder eines ihrer Salze und N,N-Dimethylformamid als organisches Lösemittel bei Temperaturen im Bereich von 100 °C bis 200 °C unter Rühren, wobei die Summe der Anteile an der Aluminiumverbindung und der 2,6-Naphthalindicarbonsäure oder einem ihrer Salze weniger als 20 Gew.-% bezogen auf das Gesamtgewicht des Reaktionsgemisches beträgt.

2. Verfahren nach Anspruch 1, **dadurch gekennzeichnet, dass** das molare Verhältnis Aluminiumverbindung zu 2,6-Naphthalindicarbonsäure oder einem ihrer Salze im Bereich von 0,3 bis 1,5 liegt.

3. Poröses metallorganisches Gerüstmaterial erhältlich nach einem Verfahren nach Anspruch 1 oder 2.

4. Gerüstmaterial nach Anspruch 3, **dadurch gekennzeichnet, dass** der erste Reflex im Bereich von 6,7° < 2θ < 7,3° und der zweite Reflex im Bereich von 13,9° < 2θ < 14,8° liegt

5. Gerüstmaterial nach Anspruch 3 oder 4, **dadurch gekennzeichnet, dass** das Gerüstmaterial als Pulver eine spezifische Oberfläche bestimmt nach Langmuir durch N₂ Adsorption bei 77 K von mindestens 1000 m²/g aufweist.

6. Formkörper enthaltend ein poröses metallorganisches Gerüstmaterial nach einem der Ansprüche 3 bis 5.

7. Formkörper nach Anspruch 6, **dadurch gekennzeichnet, dass** der Formkörper eine spezifische Oberfläche bestimmt nach Langmuir durch N₂ Adsorption bei 77 K von mindestens 500 m²/g aufweist.

8. Verwendung eines metallorganischen Gerüstmaterials nach einem der Ansprüche 3 bis 5 oder einem Formkörper nach Anspruch 6 oder 7 zur Aufnahme mindestens eines Stoffes zu dessen Speicherung, Abtrennung, kontrollierten Abgabe, chemischen Umsetzung oder als Träger.

9. Verwendung nach Anspruch 8, **dadurch gekennzeichnet, dass** der mindestens eine Stoff Kohlendioxid ist, um dieses aus einem Kohlendioxid-haltigen Gasgemisch zu trennen.

10. Verwendung nach Anspruch 8, **dadurch gekennzeichnet, dass** der mindestens eine Stoff ein Gas ist, um dieses bei einem Mindestdruck von 100 bar (absolut) zu speichern.

## Claims

1. A process for producing a porous metal-organic framework material comprising a bidentate organic compound bound by coordination to a metal ion, the metal ion being Al^{III} and the bidentate organic compound being 2,6-naphthalenedicarboxylate, wherein the X-ray diffractogram (XRD) of the framework material has a first reflection in the range from 6.5° < 2Θ < 7.5° and a second reflection in the range from 13.8° < 2Θ < 15.0°, the area of the first reflection being greatest in relation to the area of all reflections in the range from 2° < 2Θ < 70° and the area of the second reflection being second greatest, and the sum of the areas of the first and second reflections giving at least 50% in relation to the total area of all reflections in the range from 2° < 2Θ < 70°, comprising the step
- reacting a reaction mixture comprising an aluminum compound, 2,6-naphthalenedicarboxylic acid or one of its salts and N,N-dimethylformamide as organic solvent at temperatures in the range from 100°C to 200°C with stirring, the sum of the fractions of the aluminum compound and the 2,6-naphthalenedicarboxylic acid or one of its salts being less than 20% by weight based on the total weight of the reaction mixture.

2. The process according to claim 1, wherein the molar ratio of aluminum compound to 2,6-naphthalenedicarboxylic acid or one of its salts is in the range from 0.3 to 1.5.

3. A porous metal-organic framework material obtainable by a process according to claim 1 or 2.

4. The framework material according to claim 3, wherein the first reflection is in the range from 6.7° < 2Θ < 7.3° and the second reflection is in the range from 13.9° < 2Θ < 14.8°.

5. The framework material according to claim 3 or 4, wherein the framework material, as powder, has a specific surface area of at least 1000 m²/g determined according to Langmuir by N₂ adsorption at 77 K.

6. A shaped body comprising a porous metal-organic framework material according to any of claims 3 to 5.

7. The shaped body according to claim 6, wherein the shaped body has a specific surface area of at least 500 m²/g determined according to Langmuir by N₂ adsorption at 77 K.

8. The use of a metal-organic framework material according to any of claims 3 to 5 or a shaped body according to claim 6 or 7 for receiving at least one substance for its storage, separation, controlled release, chemical reaction or as support.

9. The use according to claim 8, wherein the at least one substance is carbon dioxide in order to separate it from a carbon dioxide-containing gas mixture.

10. The use according to claim 8, wherein the at least one substance is a gas in order to store it at a minimum pressure of 100 bar (absolute).

## Revendications

1. Procédé pour la réalisation d'un matériau squelette métallo-organique poreux, contenant un composé organique bidentate, lié par coordination à un ion métallique, l'ion métallique étant Al^{III} et le composé organique bidentate étant le 2,6-naphtalènedicarboxylate, **caractérisé en ce que** le diffractogramme des rayons X (XRD) du matériau squelette présente, dans la plage de 6,5° < 2θ < 7,5°, une première réflexion et, dans la plage de 13,8° < 2θ < 15,0°, une deuxième réflexion, la surface de la première réflexion étant la plus grande et la surface de la deuxième réflexion étant la deuxième dans l'ordre de grandeur par rapport à toutes les réflexions dans la plage de 2° < 2θ < 70° et la somme des surfaces de la première et de la deuxième réflexion par rapport à la surface totale de toutes les réflexions dans la plage de 2° < 2θ < 70° valant au moins 50%, comprenant l'étape de
- transformation d'un mélange réactionnel contenant un composé d'aluminium, de l'acide 2,6-naphtalènedicarboxylique ou un de ses sels et du N,N-diméthylformamide comme solvant organique à des températures dans la plage de 100°C à 200°C sous agitation, la somme des proportions de composé d'aluminium et d'acide 2,6-naphtalènedicarboxylique ou d'un de ses sels étant inférieure à 20% en poids par rapport au poids total du mélange réactionnel.

2. Procédé selon la revendication 1, **caractérisé en ce que** le rapport molaire de composé d'aluminium à acide 2,6-naphtalènedicarboxylique ou à un de ses sels se situe dans la plage de 0,3 à 1,5.

3. Matériau squelette métallo-organique poreux, pouvant être obtenu selon un procédé selon la revendication 1 ou 2.

4. Matériau squelette selon la revendication 3, **caractérisé en ce que** la première réflexion se situe dans la plage de 6,7° < 2θ < 7,3° et la deuxième réflexion se situe dans la plage de 13,9° < 2θ < 14,8°.

5. Matériau squelette selon la revendication 3 ou 4, **caractérisé en ce que** le matériau squelette présente, comme poudre, une surface spécifique déterminée selon Langmuir par adsorption de N₂ à 77 K d'au moins 1000 m²/g.

6. Corps façonné contenant un matériau squelette métallo-organique poreux selon l'une quelconque des revendications 3 à 5.

7. Corps façonné selon la revendication 6, **caractérisé en ce que** le corps façonné présente une surface spécifique déterminée selon Langmuir par adsorption de N₂ à 77 K d'au moins 500 m²/g.

8. Utilisation d'un matériau squelette métallo-organique selon l'une quelconque des revendications 3 à 5 ou d'un corps façonné selon la revendication 6 ou 7 pour l'absorption d'au moins une substance en vue de son accumulation, de sa séparation, de sa distribution contrôlée, de sa transformation chimique ou comme support.

9. Utilisation selon la revendication 8, **caractérisée en ce que** ladite au moins une substance est le dioxyde de carbone, pour séparer celui-ci d'un mélange gazeux contenant du dioxyde de carbone.

10. Utilisation selon la revendication 8, **caractérisée en ce que** ladite au moins une substance est un gaz, afin d'accumuler celui-ci à une pression minimale de 100 bars (absolus).
